# EUROPÄISCHE PATENTSCHRIFT

(11) **EP 4 312 939 B1**
(45) Veröffentlichungstag und Bekanntmachung des Hinweises auf die Patenterteilung: **03.12.2025**
(21) Anmeldenummer: 22717353.1
(22) Anmeldetag: 25.02.2022
(51) Int. Cl.: A61H 7/00

(54) **VORRICHTUNG ZUR PHYSIOTHERAPEUTISCHEN BEHANDLUNG DES MENSCHLICHEN KÖRPERS BESTEHEND AUS ZWEI SCHEIBEN MIT EINEM DAZWISCHENLIEGENDEN KINESIOLOGISCHEN BAND UND VERFAHREN ZUR PHYSIOTHERAPEUTISCHEN BEHANDLUNG DES MENSCHLICHEN KÖRPERS**
DEVICE FOR THE PHYSIOTHERAPEUTIC TREATMENT OF THE HUMAN BODY, CONSISTING OF TWO DISCS AND A KINESIOLOGY TAPE BETWEEN SAME, AND METHOD FOR THE PHYSIOTHERAPEUTIC TREATMENT OF THE HUMAN BODY
DISPOSITIF DE TRAITEMENT PHYSIOTHÉRAPEUTIQUE DU CORPS HUMAIN CONSTITUÉ DE DEUX DISQUES ET D'UNE BANDE DE KINÉSIOLOGIE ENTRE CEUX-CI, ET PROCÉDÉ DE TRAITEMENT PHYSIOTHÉRAPEUTIQUE DU CORPS HUMAIN

(30) Priorität: 03.04.2021 DE 202021001254 U
(43) Veröffentlichungstag der Anmeldung: 07.02.2024
(73) Patentinhaber: ProCure med GmbH, 63456 Hanau (DE)
(72) Erfinder: WINTER, Jan Niclas, 63110 Rodgau (DE)
(74) Vertreter: Meyer-Dulheuer MD Legal Patentanwälte PartG mbB
(86) Internationale Anmeldenummer: PCT/DE2022/100159
(87) Internationale Veröffentlichungsnummer: WO 2022/207032

(56) Entgegenhaltungen:
- EP-B1- 2 524 683
- JP-B1- 5 957 643
- NO-B1- 342 951
- US-A- 2 070 727
- US-A1- 2013 061 377

## Beschreibung

Die Erfindung richtet sich auf eine Vorrichtung zur physiotherapeutischen Behandlung des menschlichen Körpers, wobei die Vorrichtung aus zwei runden Scheiben besteht, die über einen Verankerungsstift in der ersten runden Scheibe und eine Aushöhlung in der zweiten runden Scheibe zusammensetzbar sind, und die zweite runde Scheibe auf der Unterseite eine kegelförmige Ausbuchtung besitzt, die bei der Behandlung auf der menschlichen Haut aufliegt und dadurch bei Anwendung von Druck auf die erste runde Scheibe den Druck auf die Haut über die zweite runde Scheibe punktuell aufbringt und dadurch Spannungen abbaut und die Durchblutung anregt.

Bei der Behandlung des menschlichen Körpers durch Physiotherapie wird in vielen Verfahrensweisen mechanischer Druck auf die menschliche Haut und den darunterliegenden Körper angewendet. Die Anwendung von mechanischem Druck erhöht die Durchblutung in dem Bereich, in dem der Druck angewendet wird, löst Verspannungen innerhalb der Muskulatur und unterstützt den Lymphabfluss. Das einfachste Beispiel für die Anwendung von mechanischem Druck auf die menschliche Haut ist die Massage. Bei der Massage wird mit der menschlichen Hand mechanischer Druck auf die Haut und den darunterliegenden Körper angewendet, um Verspannungen der Muskulatur zu lösen und die Durchblutung anzuregen. Das Druckmuster ist dabei meist recht einfach und richtet sich nach der Kraft und Geschicklichkeit des Masseurs. Oftmals sind jedoch in vielen Bereichen des menschlichen Körpers die Problemstellen nur in einem kleinen und eng umgrenzten Bereich angesiedelt. So ist es beispielsweise möglich, dass sich Schmerzen und Durchblutungsstörungen nur an bestimmten, eng umgrenzten Stellen des menschlichen Körpers herausbilden. Eine großflächige Behandlung ist dann oftmals nicht erwünscht, weil die Behandlung an den eigentlichen Schmerzstellen zu schwach ist und die schmerzfreien Stellen am Körper unerwünschtem mechanischen Druck ausgesetzt werden.

Ein Beispiel für Problemstellen im Körper, die in nur einem kleinen und eng umgrenzten Bereich angesiedelt sind, sind die sogenannten Triggerpunkte. Triggerpunkte sind kleine Verspannungen innerhalb der Muskulatur, die mit einer Minderdurchblutung in diesem Bereich einhergehen und bei Aktivität Schmerzen verursachen können. Diese Triggerpunkte können durch verschiedene manuelle Techniken in der Physiotherapie behandelt werden. Dies sind in der Regel Druckbehandlungen. Die Druckbehandlung muss dann genau in dem Bereich des Triggerpunktes durchgeführt werden, damit die Verspannungen innerhalb der Muskulatur abgebaut werden und die Durchblutung auch punktuell angeregt wird. Muskelverspannungen können sich oftmals auch zu sogenannten Myogelosen erweitern, bei denen die betroffenen Muskeln verhärten. Diese Myogelosen sind in der Regel sehr schmerzhaft. Auch Myogelosen und Muskelverspannungen sollten nur in dem betroffenen Bereich behandelt werden.

Eine Massage, die mit der Hand durchgeführt wird, ist oftmals nur unzureichend in der Lage, die gewünschten Ergebnisse zu erzielen. Zum einen ist die Anwendung von mechanischem Druck mit der Hand von der Physiognomie und der Muskelstärkeverteilung des Masseurs abhängig, zum anderem aber ist eine engumgrenzte Anwendung von mechanischem Druck, beispielsweise bei der Behandlung von Triggerpunkten, aufgrund der Dimensionierung der menschlichen Hand oftmals nicht möglich. Es gibt deshalb im Stand der Technik vielerlei Hilfsmittel, um eine gezieltere Anwendung von mechanischem Druck auf die menschliche Haut zu ermöglichen, als dies durch die menschliche Hand möglich ist.

Das Dokument EP0199872A1 beschreibt ein magnetisches therapeutisches Instrument, welches einen permanenten Magneten, metallische Bänder, die an ihrem rückwärtigen Ende jeweils mit der Rückseite des permanenten Magneten verbunden sind, ein Klebeband, welches auf dem permanenten Magneten aufliegt und ein abnehmbares Blatt Papier, welches auf der dem permanenten Magneten gegenüberliegenden Seite des Klebebands auf dem Klebeband aufliegt, und eine Schicht aus Germanium, die auf der dem Klebeband gegenüberliegenden Seite des permanenten Magneten aufgebracht ist und 0.05 bis 30 µm dick ist, aufweist, wobei der permanente Magnet mit der N-Polarität zu der Germaniumschicht hin orientiert ist und eine Magnetfeldstärke von 750 bis 850 Oersted aufweist, und die Germaniumschicht so ausgerichtet ist, dass die C-Achse mit der Magnetachse auf einer Linie liegt. Zur therapeutischen Behandlung des menschlichen Körpers wird das magnetische therapeutische Instrument mit der Germaniumschicht auf die Haut des menschlichen Körpers aufgebracht, wodurch unter Abnutzung der Germaniumschicht ein elektrischer Strom durch den menschlichen Körper fließt und dadurch Schmerzen und Steifheit in den Gliedern und insbesondere in der Schulter verringert werden.

Das Dokument CN213941156U beschreibt ein Pflaster zum Auflegen auf das Gesicht eines Patienten mit Schlafstörungen, wobei das Pflaster eine Pilzform aufweist und der breite, horizontale obere Teil des Pflasters zum Auflegen auf die Stirn vorgesehen ist und der schmale untere Teil zum Auflegen auf den Nasenbereich vorgesehen ist, und senkrecht zu dem Pflaster in dem Übergangsbereich von dem oberen breiten Teil zu dem unteren schmalen Teil ein Druckstab vorgesehen ist, welcher zum Aufpressen des Pflasters auf den sogenannten Yintang-Punkt dient, wodurch nach einer Anwendung über Nacht die Konzentrationsfähigkeit gestärkt und die Schlaflosigkeit beseitigt werden.

Eine weitere Offenbarung im Stand der Technik ist das Dokument NO342951B1.

Dieser Stand der Technik beschreibt Vorrichtungen, die anstelle der menschlichen Hand zur Verringerung von Schmerzen oder anderen körperlichen Beschwerden auf die Haut des Patienten aufgelegt werden. Diese Vorrichtungen wenden elektrischen Strom auf die Haut oder einen punktuellen mechanischen Druck auf eine genau vorbestimmte Stelle der Haut an. Ein punktueller Druck auf die Haut, noch dazu auf eine genau vorbestimmte Stelle unabhängig von der Stelle des auftretenden Schmerzes, ist bei einer physiotherapeutischen Behandlung oftmals nicht erwünscht. Die Schmerzen und die Beschwerden, die physiotherapeutisch behandelbar sind, sind in der Regel nicht genau punktuell verteilt, sondern erstrecken sich über einen kleinen und eng umgrenzten Bereich der Haut und des darunterliegenden Körpers. Außerdem können diese an jeder beliebigen Stelle des menschlichen Körpers auftreten.

Daher wäre es wünschenswert, über eine Vorrichtung zu verfügen, welche bei einer Physiotherapie den mechanischen Druck der Hand aufnimmt und diesen über eine bestimmte Fläche der Haut und des darunterliegenden menschlichen Körpers verteilt. Diese Vorrichtung sollte mechanischen Druck der Hand nicht punktförmig auf die menschliche Haut verteilen, sondern über einen eng umgrenzten Bereich der Haut und des darunterliegenden Körpers. Wünschenswert wäre eine bestimmte Topologie der Vorrichtung, die bedingt durch diese Topologie die Haut und den darunterliegenden Körper mechanisch anregt. Diese Vorrichtung sollte den mechanischen Druck auch möglichst auf einen genau definierten eng umgrenzten Bereich der Haut ausüben, um den dort vorhandenen Schmerz zu bekämpfen. Diese Vorrichtung sollte auch an jeder beliebigen Stelle des Körpers einsetzbar sein.

Eine Vorrichtung, die den mechanischen Druck der Hand des Masseurs aufnimmt und diesen an einer beliebigen Stelle auf die Haut des Patienten überträgt, ist im Stand der Technik nicht vorhanden. Es besteht deshalb die Aufgabe, eine Vorrichtung zur Übertragung von mechanischem Druck von der Hand eines Masseurs auf die menschliche Haut zur Verfügung zu stellen, durch die ein eng umgrenzter Bereich der menschlichen Haut behandelt wird und die bedingt durch ihre Topologie den behandelten Bereich der Haut und den darunterliegenden Körper mechanisch anregt.

Die vorliegende Erfindung löst diese Aufgabe durch eine Vorrichtung, die zusammengesetzt ist aus einer ersten runden Scheibe mit einer planaren Unterseite und einer nach außen hervorgewölbten Oberseite, und einer zweiten runden Scheibe mit einer planaren Oberseite und einer nach außen hervorgewölbten Unterseite.

Die Oberseite der ersten runden Scheibe weist dabei eine Neigung auf, deren Wölbung zum Scheibenmittelpunkt durchgängig abnimmt. Dadurch ergibt sich insgesamt eine halbkugelförmige Wölbung der Oberseite. Die halbkugelförmige Wölbung ist in der Regel abgeflacht, um ein gutes Anliegen der Hand des Masseurs zu ermöglichen. Die halbkugelförmige Wölbung der Oberseite der ersten runden Scheibe kann auch Formgebungen zur Aufnahme der Hand des Masseurs oder Anwenders aufweisen. Die Unterseite der zweiten runden Scheibe weist hingegen eine Neigung auf, deren Wölbung zuerst zunimmt und dann zum Scheibenmittelpunkt abnimmt. Dadurch ergibt sich insgesamt eine kegelförmige Wölbung der Unterseite. Durch die kegelförmige Wölbung der Unterseite der zweiten runden Scheibe steht eine Topologie der Vorrichtung zur Verfügung, die eine gute mechanische Anregung der Haut und des darunterliegenden Körpers ermöglicht. Die begrenzte Ausdehnung der Vorrichtung wiederum ermöglicht eine gute Behandlung des eng umgrenzten Bereichs, in dem die Schmerzen und die Beschwerden auftreten. Die erste und die zweite Scheibe sind dabei rund, können jedoch auch näherungsweise rund sein, so dass diese ihren Zweck einer in der Fläche gleichmäßigen Behandlung noch erfüllen.

Die erste runde Scheibe weist einen Verankerungsstift auf, der am unteren Ende eine Verdickung aufweist, und der in eine dafür vorgesehene in die zweite runde Scheibe hineinragende Aushöhlung einrastbar ist. Diese Einrastung ist wieder lösbar, um ein Auseinandernehmen und Lagern der Vorrichtung zu ermöglichen. Die erste runde Scheibe und die zweite runde Scheibe lassen sich auf diese Weise zusammenfügen. Eine physiotherapeutische Behandlung des menschlichen Körpers im Rahmen der vorliegenden Erfindung findet nur in zusammengesetztem Zustand der Vorrichtung statt, wenn die erste runde Scheibe und die zweite runde Scheibe zusammengefügt sind und der Verankerungsstift eingerastet ist.

Zwischen der ersten runden Scheibe und der zweiten runden Scheibe befindet sich ein kinesiologisches Band. Dieses dient zur Fixierung der Vorrichtung auf der Haut während der Behandlung. Dadurch lässt sich genau der Bereich behandeln, in dem Schmerzen und Beschwerden auftreten.

Beansprucht wird insbesondere eine Vorrichtung zur physiotherapeutischen Behandlung des menschlichen Körpers, umfassend
- eine erste runde Scheibe mit einer Unterseite und einer nach außen hervorgewölbten Oberseite, wobei die Neigung der Wölbung der Oberseite zum Scheibenmittelpunkt durchgängig abnimmt, und
- eine zweite runde Scheibe mit einer Oberseite und einer nach außen hervorgewölbten Unterseite, wobei die Neigung der Wölbung der Unterseite zuerst zunimmt und dann zum Scheibenmittelpunkt abnimmt,
und welche dadurch gekennzeichnet ist, dass
- die erste runde Scheibe einen zur Unterseite hin aus der Unterseite hinausragenden Verankerungsstift aufweist, welcher eine Verdickung besitzt, und
- die zweite runde Scheibe eine zur Oberseite hin in die zweite runde Scheibe hineinragende Aushöhlung aufweist, welche sich in der zweiten runden Scheibe aufweitet und welche an der Öffnung der Aushöhlung dehnbar ist und dort einen kleineren Durchmesser aufweist, als das Innere der Aushöhlung, wodurch der Verankerungsstift der ersten runden Scheibe mit der Verdickung in der Aushöhlung der zweiten runden Scheibe einrastbar ist, und
- ein kinesiologisches Band, welches eine Öffnung aufweist und welches auf den Verankerungsstift aufziehbar ist.

In einer Ausführungsform der Erfindung sind die Unterseite der ersten runden Scheibe und die Oberseite der zweiten runden Scheibe planar. Dadurch greifen die Unterseite der ersten runden Scheibe und die Oberseite der zweiten runden Scheibe mit einem dazwischenliegenden kinesiologischen Band gut ineinander. Es ist auch möglich, die Unterseite der ersten runden Scheibe und die Oberseite der zweiten runden Scheibe nicht planar zu gestalten. Diese müssen dann aber so gestaltet sein, dass diese mit einem kinesiologischen Band dazwischen ineinandergreifen.

Der Verankerungsstift weist bevorzugt die Verdickung am unteren Ende und die Aushöhlung die Aufweitung zur Aufnahme der Verdickung am inneren Ende auf. Dadurch wird nach dem Einrasten ein optimaler Halt des Verankerungsstifts in der Aushöhlung gewährleistet und der Verankerungsstift ist leichter wieder lösbar.

In einer weiteren Ausführungsform der Erfindung weist die erste runde Scheibe einen Durchmesser von 10 bis 50 mm auf. Durch diese Größe wird ein gutes Anliegen der Hand des Masseurs ermöglicht.

In einer weiteren Ausführungsform der Erfindung weist die zweite runde Scheibe ebenfalls einen Durchmesser von 10 bis 50 mm auf. Durch diese Größe ist die Behandlung eines eng umgrenzten Bereichs auf der Haut und des darunterliegenden Körpers möglich.

In einer weiteren Ausführungsform der Erfindung weist die zweite runde Scheibe eine Höhe von 5 bis 15 mm auf. Durch diese Höhe ist eine gute Bearbeitungstiefe der menschlichen Haut möglich, so dass die darunterliegenden Tastnerven angesprochen werden, die Durchblutung der unter der Haut liegenden Körperteile angeregt wird, die Spannung der Muskulatur normalisiert wird und der Lymphabfluss unterstützt wird.

Die Vorrichtung liegt üblicherweise in einer Ausführungsform vor, in der ein kinesiologisches Band auf den Verankerungsstift aufgezogen wird und die erste runde Scheibe über den Verankerungsstift in die Aushöhlung der zweiten runden Scheibe eingerastet ist, wodurch die erste runde Scheibe und die zweite runde Scheibe mit dem dazwischenliegenden kinesiologischen Band eine zusammengesetzte Vorrichtung bilden. Dadurch ist die Vorrichtung zusammengesetzt für die physiotherapeutische Behandlung nutzbar.

Kinesiologische Bänder ("Kinesiologische Tapes") sind im Stand der Technik bekannt. Ein Beispiel für ein kinesiologisches Band gibt das Dokument EP2524683B1. In der vorliegenden Erfindung wird der Verankerungsstift auf das kinesiologische Band aufgezogen. Für das Aufziehen auf den Verankerungsstift wird das kinesiologische Band gestanzt, gelocht oder eingeschnitten und der Verankerungsstift durch die entstandene Stanzung, Lochung oder den Einschnitt gezogen. Anschließend wird der Verankerungsstift in die dafür vorgesehene Aushöhlung der zweiten runden Scheibe eingerastet. In einer bevorzugten Ausführungsform ist das kinesiologische Band zum Aufziehen auf den Verankerungsstift in der geometrischen Mitte gestanzt, gelocht oder eingeschnitten. In einer bevorzugten Ausführungsform handelt es sich bei der Stanzung um eine Kreuzstanzung in der geometrischen Mitte des kinesiologischen Bandes.

Beansprucht werden auch die einzelnen Bestandteile der Vorrichtung. Beansprucht wird eine erste runde Scheibe mit einer planaren Unterseite und einer nach außen hervorgewölbten Oberseite, wobei die Neigung der Wölbung der Oberseite zum Scheibenmittelpunkt durchgängig abnimmt, und welche dadurch gekennzeichnet ist, dass diese an der Unterseite einen Verankerungsstift mit einer Verdickung aufweist. Diese kann zur Zusammensetzung zu der erfindungsgemäßen Vorrichtung verwendet werden.

Beansprucht wird auch eine zweite runde Scheibe mit einer planaren Oberseite und einer nach außen hervorgewölbten Unterseite, wobei die Neigung der Wölbung der Unterseite zuerst zunimmt und dann zum Scheibenmittelpunkt abnimmt, und welche dadurch gekennzeichnet ist, dass diese zur planaren Oberseite hin eine in die zweite runde Scheibe hineinragende Aushöhlung aufweist, welche sich in der zweiten runden Scheibe aufweitet und welche an der Öffnung der Aushöhlung dehnbar ist und dort einen kleineren Durchmesser aufweist, als das Innere der Aushöhlung. Diese kann zur Zusammensetzung zu der erfindungsgemäßen Vorrichtung verwendet werden.

Beansprucht wird auch ein kinesiologisches Band, welches dadurch gekennzeichnet ist, dass dieses in der geometrischen Mitte eine Ausstanzung aufweist. Dieses wird zur Zusammensetzung zu der erfindungsgemäßen Vorrichtung verwendet.

Die oben genannte Vorrichtung kann, bevorzugt in zusammengesetzter Form mit dem dazwischenliegenden kinesiologischen Band, zu einer physiotherapeutischen Behandlung verwendet werden. Unter einer physiotherapeutischen Behandlung sind dabei alle Behandlungsformen des menschlichen Körpers zu verstehen, die durch nichtinvasive Verfahrensschritte die Heilung von Krankheiten und Beschwerden beabsichtigen. Darunter fallen auch sportorthopädische Behandlungen. Beschrieben wird ein Verfahren zur physiotherapeutischen Behandlung des menschlichen Körpers, in welchem die oben beschriebene zusammengesetzte Vorrichtung oder eine der oben beschriebenen Ausführungsformen der Vorrichtung mit der Unterseite der zweiten runden Scheibe auf die Haut des menschlichen Körpers aufgelegt wird und durch Anwendung von mechanischem Druck von außen über die erste runde Scheibe auf diesen einwirkt. Die Anwendung erfolgt in der Regel über eine kurze Behandlungszeit.

In einer Ausführungsform des Verfahrens zur physiotherapeutischen Behandlung des menschlichen Körpers erfolgt die Auflage der oben beschriebenen zusammengesetzten Vorrichtung oder einer der oben beschriebenen Ausführungsformen und die physiotherapeutische Behandlung auf einen sogenannten Triggerpunkt. Auch ist es möglich, eine physiotherapeutische Behandlung durch Auflage der oben beschriebenen zusammengesetzten Vorrichtung oder einer der oben beschriebenen Ausführungsformen auf eine Myogelose oder eine Muskelverspannung durchzuführen.

In einer weiteren Ausführungsform des Verfahrens zur physiotherapeutischen Behandlung des menschlichen Körpers wird die Behandlung über einen Zeitraum von 1 bis 5 Tagen durchgeführt. Dadurch werden der behandelte Triggerpunkt, die Myogelose oder die Muskelverspannung deaktiviert, der Schmerz gelindert und eine Spannungsnormalisierung im Muskel erzielt.

Die Erfindung besitzt den Vorteil, eine Vorrichtung zur physiotherapeutischen Behandlung des menschlichen Körpers zur Verfügung zu stellen, die mechanischen Druck auf einen genau definierten eng umgrenzten Bereich der Haut ausübt, um den dort vorhandenen Schmerz zu bekämpfen und die bedingt durch ihre Topologie einen genau definierten eng umgrenzten Bereich der Haut und den darunterliegenden Körper mechanisch anregt.

Die Erfindung wird anhand von sieben Zeichnungen weiter erläutert, wobei diese Zeichnungen nur Ausführungsformen darstellen und die Erfindung nicht auf diese Ausführungsformen beschränkt ist.

Die Zeichnung FIG. 1 zeigt die erfindungsgemäße Vorrichtung mit einer ersten runden Scheibe und einer zweiten runden Scheibe in seitlicher Ansicht. Die Zeichnung FIG. 2 zeigt eine Schnittzeichnung der erfindungsgemäßen Vorrichtung. Die Zeichnung FIG. 3 zeigt die erfindungsgemäße Vorrichtung bei einer physiotherapeutischen Behandlung in seitlicher Ansicht. Die Zeichnung FIG. 4 zeigt die erfindungsgemäße Vorrichtung mit der ersten runden Scheibe und der zweiten runden Scheibe in getrenntem Zustand in schräg seitlicher Ansicht von oben. Die Zeichnung FIG. 5 zeigt die erfindungsgemäße Vorrichtung in getrenntem Zustand mit einem kinesiologischen Band zwischen der ersten und der zweiten runden Scheibe in seitlicher Ansicht. Die Zeichnung FIG. 6 zeigt die erfindungsgemäße Vorrichtung in zusammengesetztem Zustand mit dem kinesiologischen Band in seitlicher Ansicht. Die Zeichnung FIG. 7 zeigt die erfindungsgemäße Vorrichtung in zusammengesetztem Zustand in seitlicher Ansicht mit den dazugehörigen Dimensionierungen.

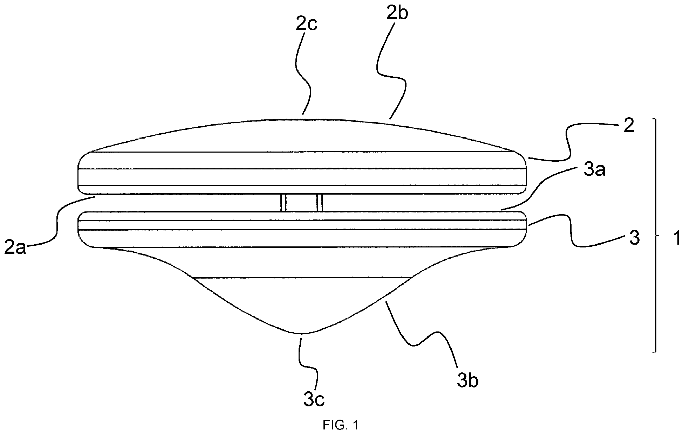

Die Zeichnung FIG. 1 zeigt die erfindungsgemäße Vorrichtung **(1)** mit einer ersten runden Scheibe **(2)** und einer zweiten runden Scheibe **(3)** in seitlicher Ansicht. Die erste runde Scheibe **(2)** besitzt eine planare Unterseite **(2a)** und eine nach außen hervorgewölbte Oberseite **(2b),** wobei die Neigung der Wölbung der Oberseite **(2b)** zum Scheibenmittelpunkt **(2c)** durchgängig abnimmt. Die zweite runde Scheibe besitzt eine planare Oberseite **(3a)** und eine nach außen hervorgewölbte Unterseite **(3b),** wobei die Neigung der Wölbung der Unterseite **(3b)** zuerst zunimmt und dann zum Scheibenmittelpunkt **(3c)** abnimmt. Dadurch weist die zweite runde Scheibe **(3)** eine kegelförmige Unterseite **(3b)** auf.

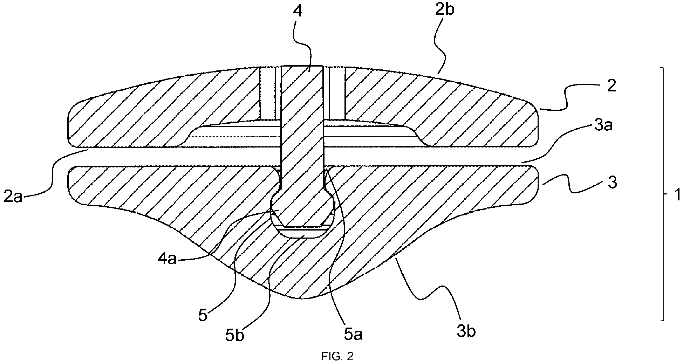

Die Zeichnung FIG. 2 zeigt eine Schnittzeichnung der erfindungsgemäßen Vorrichtung **(1)** mit der ersten runden Scheibe **(2)** und der zweiten runden Scheibe **(3),** wobei an der ersten runden Scheibe **(2)** der Verankerungsstift **(4)** zu sehen ist, der am unteren Ende eine Verdickung **(4a)** aufweist, und der in eine dafür vorgesehene in die zweite runde Scheibe **(3)** hineinragende Aushöhlung **(5)** wiederlösbar einrastbar ist. Die Aushöhlung **(5)** ist an der Öffnung **(5a)** dehnbar, weist dort einen kleineren Durchmesser auf als das Innere der Aushöhlung **(5)** und weitet sich zum inneren Ende hin auf **(5b).** Dadurch ist der Verankerungsstift **(4)** der ersten runden Scheibe **(2)** mit der Verdickung **(4a)** am inneren Ende in der Aufweitung **(5b)** der Aushöhlung **(5)** der zweiten runden Scheibe **(3)** einrastbar.

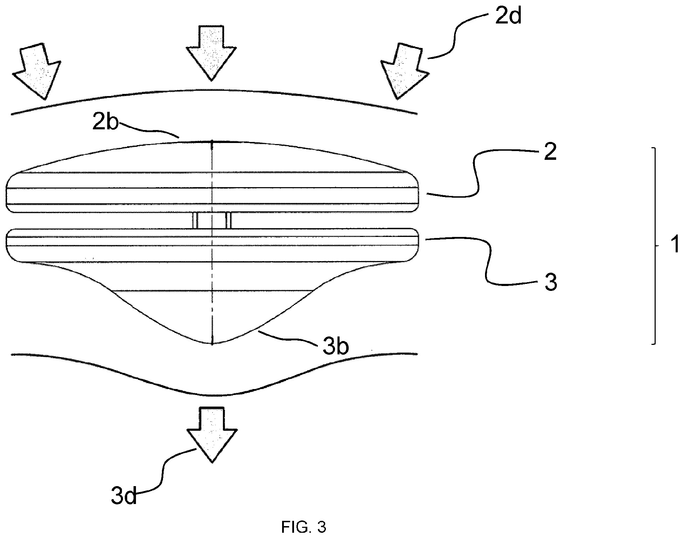

Die Zeichnung FIG. 3 zeigt die erfindungsgemäße Vorrichtung **(1)** mit der ersten runden Scheibe **(2)** und der zweiten runden Scheibe **(3)** bei einer physiotherapeutischen Behandlung in seitlicher Ansicht. Auf die hervorgewölbte Oberseite **(2b)** der ersten runden Scheibe **(2)** wird mechanischer Druck **(2d)** mit der Hand ausgeübt, so dass die kegelförmige Unterseite **(3b)** der zweiten runden Scheibe **(3)** mechanischen Druck **(3d)** auf die darunterliegende menschliche Haut ausübt. Bedingt durch die kegelförmige Topologie der Unterseite **(3b)** der zweiten runden Scheibe **(3)** werden die Haut und der darunterliegende Körper mechanisch angeregt.

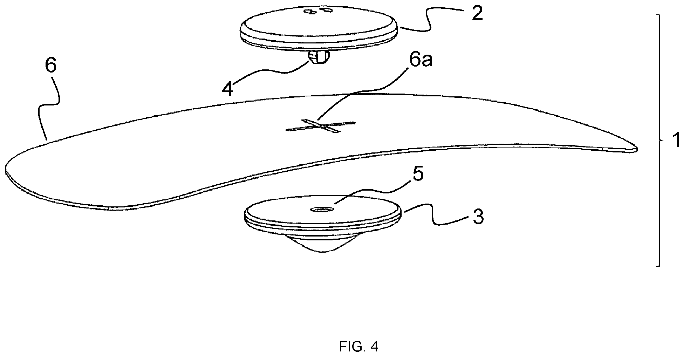

Die Zeichnung FIG. 4 zeigt die erfindungsgemäße Vorrichtung **(1)** mit der ersten runden Scheibe **(2)** und der zweiten runden Scheibe **(3)** in getrenntem Zustand in schräg seitlicher Ansicht von oben. Zwischen der ersten runden Scheibe **(2)** und der zweiten runden Scheibe **(3)** ist ein kinesiologisches Band **(6)** angeordnet. Zu sehen sind auch der Verankerungsstift **(4)** und die in die zweite runde Scheibe **(3)** hineinragende Aushöhlung **(5).** Das kinesiologische Band **(6)** weist in der geometrischen Mitte eine kreuzförmige Ausstanzung **(6a)** auf, durch die der Verankerungsstift **(4)** gezogen und die erste runde Scheibe **(2)** und die zweite runde Scheibe **(3)** mit dem dazwischenliegenden kinesiologischen Band **(6)** zur erfindungsgemäßen Vorrichtung **(1)** zusammengesetzt werden können.

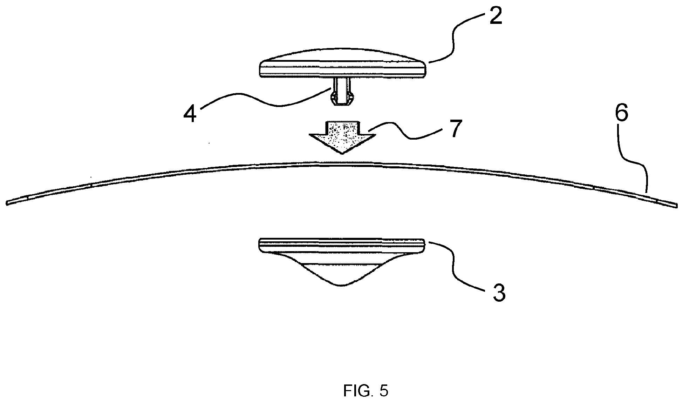

Die Zeichnung FIG. 5 zeigt die erfindungsgemäße Vorrichtung **(1)** mit der ersten runden Scheibe **(2)** und der zweiten runden Scheibe **(3)** in getrenntem Zustand mit dem kinesiologischen Band **(6)** zwischen der ersten **(2)** und der zweiten runden Scheibe **(3)** in seitlicher Ansicht. Die erste runde Scheibe **(2)** wird über den Verankerungsstift **(4)** in die zweite runde Scheibe **(3)** eingesetzt **(7),** wobei das kinesiologische Band **(6)** zwischen die erste runde Scheibe **(2)** und die zweite runde Scheibe **(3)** eingesetzt wird und der Verankerungsstift **(4)** über die Ausstanzung **(6a)** durch das kinesiologische Band **(6)** geführt wird.

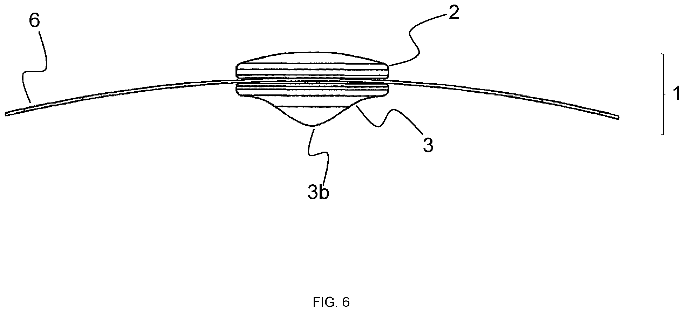

Die Zeichnung FIG. 6 zeigt die erfindungsgemäße Vorrichtung **(1)** mit der ersten runden Scheibe **(2)** und der zweiten runden Scheibe **(3)** in zusammengesetztem Zustand mit dem kinesiologischen Band **(6)** zwischen der ersten (2) und der zweiten runden Scheibe **(3)** in seitlicher Ansicht. Zur Anwendung in der physiotherapeutischen Behandlung wird die kegelförmige Unterseite **(3b)** auf die zu behandelnde menschliche Haut aufgelegt und über das kinesiologische Band **(6)** auf der menschlichen Haut befestigt.

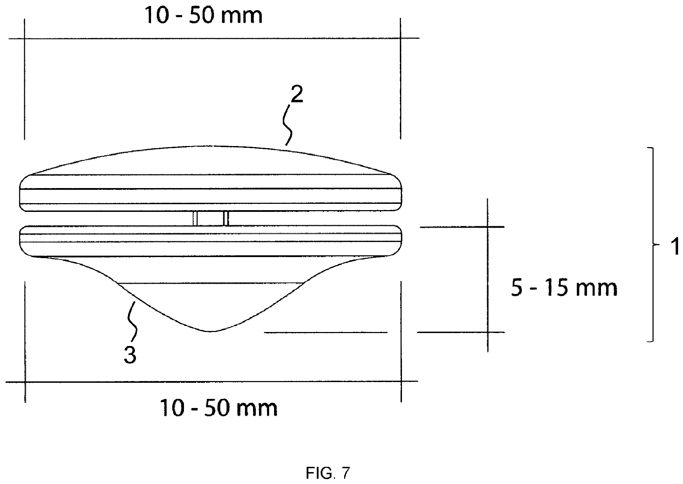

Die Zeichnung FIG. 7 zeigt die erfindungsgemäße Vorrichtung **(1)** mit der ersten runden Scheibe **(2)** und der zweiten runden Scheibe **(3)** in zusammengesetztem Zustand in seitlicher Ansicht mit den dazugehörigen Dimensionierungen. Die erste runde Scheibe **(2)** und die zweite runde Scheibe **(3)** weisen einen Durchmesser von jeweils 10 bis 50 mm auf. Die zweite runde Scheibe **(3)** weist eine Höhe von 5 bis 15 mm auf.

Bezugszeichenliste
- 1: Vorrichtung
- 2: Erste runde Scheibe
- 2a: Unterseite der ersten runden Scheibe
- 2b: Oberseite der ersten runden Scheibe
- 2c: Scheibenmittelpunkt der ersten runden Scheibe
- 2d: Mechanischer Druck der Hand
- 3: Zweite runde Scheibe
- 3a: Oberseite der zweiten runden Scheibe
- 3b: Unterseite der zweiten runden Scheibe
- 3c: Scheibenmittelpunkt der zweiten runden Scheibe
- 3d: Mechanischer Druck auf die menschliche Haut
- 4: Verankerungsstift
- 4a: Verdickung
- 5: Aushöhlung
- 5a: Öffnung
- 5b: Aufweitung
- 6: Kinesiologisches Band
- 6a: Kreuzförmige Ausstanzung
- 7: Einsetzungsvorgang

## Patentansprüche

1. Vorrichtung (1) zur physiotherapeutischen Behandlung des menschlichen Körpers, umfassend
• eine erste runde Scheibe (2) mit einer Unterseite (2a) und einer nach außen hervorgewölbten Oberseite (2b), wobei die Neigung der Wölbung der Oberseite (2b) zum Scheibenmittelpunkt (2c) durchgängig abnimmt, und
• ein kinesiologisches Band (6),
**dadurch gekennzeichnet, dass**
• die Vorrichtung (1) eine zweite runde Scheibe (3) mit einer Oberseite (3a) und einer nach außen hervorgewölbten Unterseite (3b) umfasst, wobei die Neigung der Wölbung der Unterseite (3b) zuerst zunimmt und dann zum Scheibenmittelpunkt (3c) abnimmt, und
• die erste runde Scheibe (2) einen zur Unterseite (2a) hin aus der Unterseite (2a) hinausragenden Verankerungsstift (4) aufweist, welcher eine Verdickung (4a) besitzt, und
• die zweite runde Scheibe (3) eine zur Oberseite (3a) hin in die zweite runde Scheibe (3) hineinragende Aushöhlung (5) aufweist, welche sich in der zweiten runden Scheibe (3) aufweitet und welche an der Öffnung der Aushöhlung (5) dehnbar ist und dort einen kleineren Durchmesser aufweist, als das Innere der Aushöhlung (5), wodurch der Verankerungsstift (4) der ersten runden Scheibe (2) mit der Verdickung (4a) in der Aushöhlung (5) der zweiten runden Scheibe (3) einrastbar ist, und
• das kinesiologische Band (6) eine Öffnung (6a) aufweist und auf den Verankerungsstift (4) aufziehbar ist.

2. Vorrichtung (1) gemäß Patentanspruch 1, **dadurch gekennzeichnet, dass** die Unterseite (2a) der ersten runden Scheibe (3) und die Oberseite der zweiten runden Scheibe planar sind.

3. Vorrichtung (1) gemäß einem der Patentansprüche 1 oder 2, **dadurch gekennzeichnet, dass** der Verankerungsstift (4) die Verdickung (4a) am unteren Ende aufweist und die Aushöhlung (5) am inneren Ende eine Aufweitung (5b) zur Aufnahme der Verdickung (4a) aufweist.

4. Vorrichtung (1) gemäß einem der Patentansprüche 1 bis 3, **dadurch gekennzeichnet, dass** die erste runde Scheibe (2) einen Durchmesser von 10 bis 50 mm aufweist.

5. Vorrichtung (1) gemäß einem der Patentansprüche 1 bis 4, **dadurch gekennzeichnet, dass** die zweite runde Scheibe (3) einen Durchmesser von 10 bis 50 mm aufweist.

6. Vorrichtung (1) gemäß einem der Patentansprüche 1 bis 5, **dadurch gekennzeichnet, dass** die zweite runde Scheibe (3) eine Höhe von 5 bis 15 mm aufweist.

7. Vorrichtung (1) gemäß einem der Patentansprüche 1 bis 6, **dadurch gekennzeichnet, dass** das kinesiologische Band (6) auf den Verankerungsstift (4) aufgezogen wird und die erste runde Scheibe (2) über den Verankerungsstift (4) in die Aushöhlung (5) der zweiten runden Scheibe (3) eingerastet ist, wodurch die erste runde Scheibe (2) und die zweite runde Scheibe (3) mit dem dazwischenliegenden kinesiologischen Band (6) eine zusammengesetzte Vorrichtung (1) bilden.

8. Vorrichtung (1) gemäß einem der Patentansprüche 1 bis 7, **dadurch gekennzeichnet, dass** ein Bestandteil der Vorrichtung (1) eine erste runde Scheibe (2) mit einer Unterseite (2a) und einer nach außen hervorgewölbten Oberseite (2b) ist, wobei die Neigung der Wölbung der Oberseite der ersten runden Scheibe (2) zum Scheibenmittelpunkt (2c) durchgängig abnimmt, und die erste runde Scheibe (2) an der Unterseite (2a) einen Verankerungsstift (4) mit einer Verdickung (4a) aufweist, und die erste runde Scheibe (2) zur Zusammensetzung zu der Vorrichtung (1) verwendbar ist.

9. Vorrichtung (1) gemäß einem der Patentansprüche 1 bis 8, **dadurch gekennzeichnet, dass** ein Bestandteil der Vorrichtung (1) eine zweite runde Scheibe (3) mit einer Oberseite (3a) und einer nach außen hervorgewölbten Unterseite (3b) ist, wobei die Neigung der Wölbung der Unterseite der zweiten runden Scheibe (3) zuerst zunimmt und dann zum Scheibenmittelpunkt (3c) abnimmt, und die zweite runde Scheibe (3) zur Oberseite (3a) hin eine in die zweite runde Scheibe (3) hineinragende Aushöhlung (5) aufweist, welche sich in der zweiten runden Scheibe (3) aufweitet und welche an der Öffnung der Aushöhlung (5) dehnbar ist und dort einen kleineren Durchmesser aufweist als das Innere der Aushöhlung (5), und die zweite runde Scheibe (3) zur Zusammensetzung zu der Vorrichtung (1) verwendbar ist.

10. Vorrichtung (1) gemäß einem der Patentansprüche 1 bis 9, **dadurch gekennzeichnet, dass** ein Bestandteil der Vorrichtung (1) ein kinesiologisches Band (6) ist, und dieses kinesiologische Band (6) in der geometrischen Mitte eine Ausstanzung (6a) aufweist, und zur Zusammensetzung zu der Vorrichtung (1) verwendbar ist.

## Claims

1. Apparatus (1) for the physiotherapeutic treatment of the human body, comprising
• a first round disc (2) with an underside (2a) and an outwardly bulging upper side (2b), whereby the slope of the curvature of the upper side (2b) decreases consistently towards the centre of the disc (2c), and
• a kinesiology tape (6),
**characterised in that**
• the apparatus (1) comprises a second round disc (3) with an upper side (3a) and an outwardly bulging underside (3b), whereby the slope of the curvature of the underside (3b) initially increases and then decreases towards the centre of the disc (3c), and
• the first round disc (2) has an anchoring pin (4) that protrudes from the underside (2a) towards the underside (2a), and has a thickening (4a), and
• the second round disc (3) has a hollow (5) projecting into the second round disc (3) toward the upper side (3a), and this hollow (5) expands into the second round disc (3) and is stretchable at the opening of the hollow (5) where it has a smaller diameter than the interior of the hollow (5), whereby the anchoring pin (4) of the first round disc (2) can be slotted in to the thickening (4a) in the hollow (5) of the second round disc (3), and
• the kinesiology tape (6) has an opening (6a) and can be pulled onto the anchoring pin (4).

2. Apparatus (1) according to patent claim 1, **characterised in that** the underside (2a) of the first round disc (3) and the upper side of the second round disc are planar.

3. Apparatus (1) according to one of the patent claims 1 or 2, **characterised in that** the anchoring pin (4) has the thickening (4a) at the lower end and the hollow (5) has a widening (5b) at the inner end for accommodating the thickening (4a).

4. Apparatus (1) according to one of the patent claims 1 to 3, **characterised in that** the first round disc (2) has a diameter of 10 to 50 mm.

5. Apparatus (1) according to one of the patent claims 1 to 4, **characterised in that** the second round disc (3) has a diameter of 10 to 50 mm.

6. Apparatus (1) according to one of the patent claims 1 to 5, **characterised in that** the second round disc (3) has a height of 5 to 15 mm.

7. Apparatus (1) according to one of the patent claims 1 to 6, **characterised in that** the kinesiology tape (6) is pulled onto the anchoring pin (4) and the first round disc (2) is snapped into the hollow (5) of the second round disc (3) via the anchoring pin (4), whereby the first round disc (2) and the second round disc (3) with the kinesiology tape (6) lying therebetween form a composite apparatus (1).

8. Apparatus (1) according to one of the patent claims 1 to 7, **characterised in that** a component of the apparatus (1) is a first round disc (2) with an underside (2a) and an outwardly bulging upper side (2b), wherein the slope of the curvature of the upper side of the first round disc (2) decreases consistently towards the centre of the disc (2c), and the first round disc (2) has an anchoring pin with a thickening (4a) on the underside (2a), and the first round disc (2) can be used for assembly to the apparatus (1).

9. Apparatus (1) according to one of the patent claims 1 to 8, **characterised in that** a component of the apparatus (1) is a second round disc (3) with an upper side (3a) and an outwardly bulging underside (3b), whereby the slope of the curvature of the underside of the second round disc (3) initially increases and then decreases towards the centre of the disc (3c), and the second round disc (3) has a hollow (5) that projects into the second round disc (3) towards the upper side (3a), and this hollow expands in the second round disc (3) and is stretchable at the opening of the hollow (5) and has a smaller diameter there than the interior of the hollow (5), and the second round disc (3) can be used for assembly to the apparatus (1).

10. Apparatus (1) according to one of the patent claims 1 to 9, **characterised in that** a component of the apparatus (1) is a kinesiology tape (6), and this kinesiology tape (6) has a punched-out portion (6a) in the geometric centre and can be used for assembly to the apparatus (1).

## Revendications

1. Dispositif (1) pour le traitement physiothérapeutique du corps humain, comprenant
• un premier disque rond (2) avec une face inférieure (2a) et une face supérieure (2b) bombée vers l'extérieur, l'inclinaison du bombement de la face supérieure (2b) diminuant de manière continue vers le centre du disque (2c), et
• une bande kinésiologique (6),
**caractérisé en ce que**
• le dispositif (1) comprend un deuxième disque rond (3) avec une face supérieure (3a) et une face inférieure (3b) bombée vers l'extérieur, l'inclinaison du bombement de la face inférieure (3b) augmentant d'abord et diminuant ensuite vers le centre du disque (3c), et
• que le premier disque rond (2) présente une goupille d'ancrage (4) dépassant de la face inférieure (2a) à travers la face inférieure (2a), laquelle possède un renflement (4a), et
• que le deuxième disque rond (3) présente une cavité (5) pénétrant dans le deuxième disque rond (3) vers la face supérieure (3a), qui s'élargit dans le deuxième disque rond (3) et qui est extensible à l'ouverture de la cavité (5) et présente à cet endroit un diamètre plus petit que l'intérieur de la cavité (5), ce qui permet d'encliqueter la goupille d'ancrage (4) du premier disque rond (2) avec le renflement (4a) dans la cavité (5) du deuxième disque rond (3), et
• que la bande kinésiologique (6) présente une ouverture (6a) et peut être enfilée sur la goupille d'ancrage (4).

2. Dispositif (1) selon la revendication 1, **caractérisé en ce que** la face inférieure (2a) du premier disque rond (3) et la face supérieure du deuxième disque rond sont planes.

3. Dispositif (1) selon l'une des revendications 1 ou 2, **caractérisé en ce que** la goupille d'ancrage (4) présente le renflement (4a) à l'extrémité inférieure et que la cavité (5) présente à l'extrémité intérieure un élargissement (5b) pour recevoir le renflement (4a).

4. Dispositif (1) selon l'une des revendications 1 à 3, **caractérisé en ce que** le premier disque rond (2) présente un diamètre de 10 à 50 mm.

5. Dispositif (1) selon l'une des revendications 1 à 4, **caractérisé en ce que** le deuxième disque rond (3) présente un diamètre de 10 à 50 mm.

6. Dispositif (1) selon l'une des revendications 1 à 5, **caractérisé en ce que** le deuxième disque rond (3) présente une hauteur comprise entre 5 et 15 mm.

7. Dispositif (1) selon l'une des revendications 1 à 6, **caractérisé en ce que** la bande kinésiologique (6) est enfilée sur la goupille d'ancrage (4) et que le premier disque rond (2) est enclenché dans la cavité (5) du deuxième disque rond (3) via la goupille d'ancrage (4), le premier disque rond (2) et le deuxième disque rond (3) formant ainsi un dispositif assemblé (1) avec la bande kinésiologique (6) intermédiaire.

8. Dispositif (1) selon l'une des revendications 1 à 7, **caractérisé en ce qu'un** composant du dispositif (1) est un premier disque rond (2) avec une face inférieure (2a) et une face supérieure (2b) bombée vers l'extérieur, l'inclinaison du bombement de la face supérieure du premier disque rond (2) diminuant de manière continue vers le centre du disque (2c), et que le premier disque rond (2) présente sur la face inférieure (2a) une goupille d'ancrage avec un renflement (4a), et que le premier disque rond (2) peut être utilisé pour l'assemblage avec le dispositif (1).

9. Dispositif (1) selon l'une des revendications 1 à 8, **caractérisé en ce qu'**un composant du dispositif (1) est un deuxième disque rond (3) avec une face supérieure (3a) et une face inférieure (3b) bombée vers l'extérieur, l'inclinaison du bombement de la face inférieure du deuxième disque rond (3) augmentant d'abord et diminuant ensuite vers le centre du disque (3c), et que le deuxième disque rond (3) présente une cavité (5) pénétrant dans le deuxième disque rond (3) vers la face supérieure (3a), qui s'élargit dans le deuxième disque rond (3) et qui est extensible à l'ouverture de la cavité (5) et présente à cet endroit un diamètre plus petit que l'intérieur de la cavité (5), et que le deuxième disque rond (3) peut être utilisé pour l'assemblage avec le dispositif (1).

10. Dispositif (1) selon l'une des revendications 1 à 9, **caractérisé en ce qu'un** composant du dispositif (1) est une bande kinésiologique (6), et que cette bande kinésiologique (6) présente au centre géométrique une découpe (6a), et peut être utilisée pour l'assemblage avec le dispositif (1).
